**Europäisches Patentamt**

**European Patent Office**

**Office européen des brevets**

(19)

(11) Veröffentlichungsnummer : **0 135 718 B2**

(12) ## NEUE EUROPÄISCHE PATENTSCHRIFT

(45) Veröffentlichungstag der neuen Patentschrift :
**08.07.92 Patentblatt 92/28**

(21) Anmeldenummer : **84108979.0**

(22) Anmeldetag : **28.07.84**

(51) Int. Cl.⁵ : **C07C 27/12, // C07C35/08,
C07C49/403, C07C29/50,
C07C45/33**

(54) **Verfahren zur kontinuierlichen Herstellung von Sauerstoff enthaltenden Verbindungen.**

Die Akte enthält technische Angaben, die nach dem Eingang der Anmeldung eingereicht wurden und die nicht in dieserPatentschrift enthalten sind.

(30) Priorität : **10.08.83 DE 3328771**

(43) Veröffentlichungstag der Anmeldung :
**03.04.85 Patentblatt 85/14**

(45) Bekanntmachung des Hinweises auf die Patenterteilung :
**18.06.86 Patentblatt 86/25**

(45) Bekanntmachung des Hinweises auf die Entscheidung über den Einspruch :
**08.07.92 Patentblatt 92/28**

(84) Benannte Vertragsstaaten :
**BE CH DE FR GB IT LI NL**

(56) Entgegenhaltungen :
DE-A- 1 090 659
DE-C- 1 287 575
NL-A- 6 918 883
US-A- 3 530 185
US-A- 3 755 452
US-A- 3 846 079

(56) Entgegenhaltungen :
**J.W.M. STEEMAN et al. I.E.C. 14 (1961), 139 - 150: A pilot plant study of the oxidation of cyclohexane with air underpressure**
**L.L. v. DIERENDONCK, Ph. D. THESIS, Twente University of technology, 1970: Vergrotingsregels voor gasbelwassers**

(73) Patentinhaber : **BASF Aktiengesellschaft
Carl-Bosch-Strasse 38
W-6700 Ludwigshafen (DE)**

(72) Erfinder : **Hartig, Juergen, Dr.
In der Schleit 9
W-6718 Gruenstadt (DE)**
Erfinder : **Schuch, Gunter, Dr.
Ulrich-v.-Hutten-Strasse 5
W-6700 Ludwigshafen (DE)**
Erfinder : **Stoessel, Armin, Dr.
Immengaertenweg 20
W-6710 Frankenthal (DE)**
Erfinder : **Herrmann, Guenter, Dr.
Haeuserstrasse 21
W-6900 Heidelberg (DE)**
Erfinder : **Brunner, Arthur
Hafenstrasse 40
W-6700 Ludwigshafen (DE)**
Erfinder : **Zehner, Peter, Dr.
Erich-Kaestner-Strasse 15
W-6700 Ludwigshafen (DE)**
Erfinder : **Grosskinsky, Otto-Alfred, Dr.
Semmelweisstrasse 8
W-6700 Ludwigshafen (DE)**

EP 0 135 718 B2

Jouve, 18, rue Saint-Denis, 75001 PARIS

## Beschreibung

Gegenstand der Erfindung ist ein kontinuierliches Verfahren zur Herstellung von Sauerstoff enthaltenden Verbindungen durch Oxidation von Kohlenwasserstoffen in flüssiger Phase mit molekularem Sauerstoff enthaltenden Gasen bei erhöhter Temperatur und unter erhöhtem Druck, wobei man die molekularen Sauerstoff enthaltenden Gasen an mehreren Stellen im Verlauf der Reaktionszone über Düsenöffnungen nach unten gerichtet einleitet.

Die Oxidation von Kohlenwasserstoffen mit molekularem Sauerstoff enthaltenden Gasen ist eine in der Technik veilfach ausgeübte Arbeitsweise. Hierbei ist man bestrebt, die Oxidation der Kohlenwasserstoffe nicht unkontrolliert ablaufen zu lassen sondern zu dem jeweils gewünschten Wertprodukt zu führen die Menge an Nebenprodukten zu vermindern. Eine in der Technik bedeutungsvolle Reaktion dieser Art ist die Oxidation von Cyclohexan zu Cylohexanol und Cyclohexanon. Ein solches Verfahren wird beispielsweise beschrieben in der DE-PS 1 287 575. Bei diesem Verfahren wird Cyclohexan von oben in eine Reaktionszone zugeleitet und molekularen Sauerstoff enthaltende Gase im Gegenstrom geführt. Die Reaktionszone ist durch Lochbleche, die nicht den gesamten Querschnitt bedecken, in mehrere Zonen unterteilt, wobei die molekularen Sauerstoff enthaltenden Gase jeweils nach unten unterhalb den Lochblechen zugeführt werden. Eine solche Arbeitsweise erlaubt keine gleichmäßige Zuführung von molekularem Sauerstoff und führt insbesondere zu einem ungleichmäßige Fließen des Reaktionsgemisches. Darüber hinaus führt es zur Ausbildung von Sauerstoff enthaltenden Gaspuffern, was wegen der Explosionsgefahr nicht unproblematisch ist. Insgesamt führt die dort beschriebene Arbeitsweise zu einer ungleichmäßigen Beaufschlagung mit molekularem Sauerstoff.

Es war deshalb die technische Aufgabe gestellt, ein Verfahren zur Oxidation von Kohlenwasserstoffen mit molekularem Sauerstoff enthaltenden Gasen zur Verfügung zu stellen, bei dem die Oxidation gleichmäßig verläuft, hohe Ausbeuten an Wertprodukten erzielt werden und die Menge an Nebenprodukten minimiert wird.

Diese Aufgabe wird gelöst in einem Verfahren zur kontinuierlichen Herstellung von Sauerstoff enthaltenden Verbindungen durch Oxidation von Kohlenwasserstoffen in flüssiger Phase mit molekularem Sauerstoff enthaltenden Gasen bei erhöhter Temperatur und unter erhöhtem Druck, wobei man die molekularen Sauerstoff enthaltenden Gase an mehreren Stellen im Verlauf der Reaktionszone über Düsenöffnungen nach unten gerichtet einleitet, dadurch gekennzeichnet, daß man die molekularen Sauerstoff enthaltenden Gase mit einer Austrittsgeschwindigkeit an jeder Düsenöffnung von 0,03 bis 0,3 m/sec in einer Menge von 0.001 bis 10 l/sec je Düsenöffnung in das flüssige Reaktionsgemisch einleitet und dieses über das Volumen der Reaktionszone im wesentlichen gleichmäßig mit molekularem Sauerstoff enthaltenden Gasen in Berührung bringt.

Das neue Verfahren hat den Vorteil, daß es keiner mechanischen Mittel zur Durchmischung bedarf und auf einfache Weise eine gleichmäßige Verteilung des molekularem Sauerstoffs im zu oxydierenden Kohlenwasserstoff bewirkt wird. Das neue Verfahren hat insbesondere den Vorteil, daß sich keine zusammenhängende Gasphase ausbildet. Ferner hat das neue Verfahren den Vorteil daß die Oxidation gleichmäßige verläuft; hohe Ausbeuten an Wertprodukten erzielt werden und die Menge an Nebenprodukten vermindert wird.

Erfindungsgemäß verwendet man als Ausgangsstoffe Kohlenwasserstoffe, insbesondere Alkane mit 4 bis 18 Kohlenstoffatomen. Cycloalkane mit 5 bis 12 Kohlenstoffatomen im Ring oder Alkylaromaten mit 7 bis 12 Kohlenstoffatomen. Alkane werden hierbei zu Fettsäuren, z. B. Butan zu Essigsäure, oxydiert oder Toluol zu Benzosäure. Besondere Bedeutung hat die Oxidation von Cycloalkanen zu den entsprechenden Cycloalkanolen und -alkanonen erlangt.

Die Oxidation wird mittels molekularen Sauerstoff enthaltenden Gasen durchgeführt. Vorteilhaft enthalten solche Gase 5 bis 30 Vol.% molekularen Sauerstoff. Hierbei hält man vorteilhaft Temperaturen von 100 bis 180°C und Drücke von 5 bis 30 bar ein. Druck und Temperatur werden so aufeinander abgestimmt, daß zu jedem Zeitpunkt die Umsetzung in flüssiger Phase erfolgt.

Die Umsetzung erfolgt vorteilhaft in liegenden oder insbesondere stehenden Reaktionszonen. Die molekularen Sauerstoff enthaltenden Gase werden an mehreren Stellen im Verlauf der Reaktionszone über Düsenöffnungen nach unten gerichtet in die flüssigen Kohlenwasserstoffe eingeleitet. Ein wesentliches Merkmal der Erfindung ist es, daß die molekularen Sauerstoff enthaltenden Gase mit einer Austrittsgeschwindigkeit an jeder Düsenöffnung von 0.03 bis 0.3 m/sec. in einer Menge von 0.001 bis 10 l/sec. insbesondere 0.1 bis 1.0 l/sec. je Düsenöffnung in das flüssige Reaktionsgemisch eingeleitet werden. Die Düsenöffnungen sind über das Volumen der Reaktionszone im wesentlichen gleichmäßig verteilt. Dies erzielt man beispielsweise dadurch, daß man an mehreren Stellen in im wesentlichen gleichen Abständen im Verlauf der Reaktionszone Düsenöffnungen, die jeweils über dem Querschnitt der Reaktionszone gleichmäßig verteilt sind, anordnet. Die Abstände im Verlauf der Reaktionszone entsprechen vorteilhat dem 0.5 bis 3fachen des Durchmessers der Reaktionszone und sind insbesondere so gewählt, daß in den aufsteigenden Gasblasen aus vorangehender Zufuhrstelle der molekulare Sauerstoff noch nicht vollständig. z.B. 60 bis 90% des ursprünglichen Gahalts, verbraucht ist. Hiermit erzielt man eine räumlich im wesentlichen gleichmäßige Beaufschlagung des zu oxydierenden Kohlenwasserstoffs mit molekularem Sauerstoff enthaltenden Gasen. Es versteht sich, daß über jede Düsenöffnung im wesentlichen die gleiche Gasmenge zugeführt wird. Das nicht vollständige Abreagieren des Sauerstoffs ergibt ebenfalls eine Vergleichmäßigung der $O_2$-Konzentration im Reaktor. Die $O_2$-Endkonzentration (Abgaskonzentration) wird im obersten Teil des Reaktors eingestellt.

Durch das Zuführen von molekularen Sauerstoff enthaltenden Gasen mit einer Austrittsgeschwindigkeit von 0.03 bis 0.3 m/sec an der Düsenöffnung mit einer Menge von 0.001 bis 10 l/sec je Düsenöffnung, wobei die Düsenöffnungen nach unten gerichtet sind, erzielt man Blasen mit einem Durchmesser von $\geqq$ 10 mm. z. B. von 10 bis 100 mm Durchmesser, die zunächst größer als die Gleichgewichtsblasen sind und im Verlauf der Reaktionszone in Blasen zerfallen die den Gleichgewichtsblasen entsprechen. Unter Gleichgewichtsblasen versteht man solch Blasen, die sich in einer gewissen Entfernung von der Düsenöffnung durch Zerteilungs- oder Koaleszenzvorgänge bilden. z.B. für das System Cyclohexan/Luft, ergeben sich hierbei im Mittel Durchmesser der Gleichgewichsblasen von 1 bis 10 mm.

In der technischen Ausführung verfährt man so, daß man über Zufuhrleitungen mit einer großen Zahl von sehr kleinen Bohrungen, die einem definierten Druckverlust bewirken, jede Austrittsstelle gleichmäßig mit Sauerstoff enthaltenden Gas versorgt. Aus jeder dünnen Bohrung gelangt das molekularen Sauerstoff enthaltende Gas in einen oben geschlossenen und nach unten offenen Raum der so dimensioniert ist, daß bei den angewandten Gasmengen die oben beschriebene Austrittsgeschwindigkeiten des Gases in das Reaktionsmedium erzielt werden. Zum Beispiel verfährt man so, daß man durch eine dünnere Bohrung Sauerstoff enthaltende Gase in eine unten offene und außer der dünnen Bohrung oben geschlossen Erweiterung enleitet. Die Erweiterung kann zylindrisch, kegelförmig, rechteckig bzw. quadratisch, trompetenförmig oder glockenförmig gestaltet sein. Der untere Rand der Erweiterung kann auch gezackt oder schräge sein. Die geometrischen Dimensionen dieser Erweiterung richten sich nach der einzuhaltenden Austrittsgeschwindigkeit an der Düsenöffnung und austretenden Menge an Gas und sind mit den gegebenen Daten leicht berechenbar.

Die Reaktionszonen sind vorteilhaft gekammert, d.h. in Abschnitte unterteilt, um eine Rückvermischung zu vermeiden. Bei liegenden Reaktionszonen erzielt man dies durch Überläufe oder Trennwände mit Durchlässen : bei stehenden Reaktionszonen wird dies durch in reglelmäßigen Abstanden eingebaute Lochbleche bewirkt. Bei stehenden Reaktionszonen hat es sich darüberhinaus besonders vorteilhaft erwiesen, wenn man die zu oxydierenden Kohlenwasserstoffe und die molekularen Sauerstoff enthaltenden Gase von unten nach oben im Gleichstrom leitet. Ferner hat es sich bewährt, wenn sich in der Reaktionszone keine zusammenhängende Gasphase ausbildet.

Das Verfahren nach der Erfindung sie an der Oxidation von Cycloalkanen zu einem Gemisch aus Cycloalkanolen. Cycloalkanonen und Cycloalkylhydroperoxiden, z.B. der Oxidation von Cyclohexan, näher erlautert. Man verwendet eine stehende Reaktionszone die durch Lochbleche, die in gleichmäßigen Abstanden angeordnet sind, in Kammern unterteilt ist. Die Lochbleche haben vorteilhaft einen freien Querschnitt von 3 bis 20%, insbesondere 5 bis 10%. Oberhalb jeden Lochblechs sind über den Querschnitt gleichmäßig verteilt Düsen angeordnet, wobei die Zuführungsöffnungen mit Erweiterungen versehen sind. deren Öffnung nach unten zeigt. Durch die Reaktionszone leitet man von unten nach oben Cyclohexan. Gleichzeitig führt man molekularen Sauerstoff enthaltende Gase, z.B. mit einem Sauerstoffgehalt von 1 bis 30 Vol.% durch jede Düsenöffnung in einer Menge von 0.001 bis 10 l/sec ein, wobei man eine Austrittsgeschwindigkeit an der Düsenöffnung von 0.03 bis 0.3 m/sec einhält. Die Umsetzung wird bei einer Temperatur von 120 bis 180°C und unter einem Druck von 5 bis 30 bar durchgeführt. Die Menge an zugeführten molekularen Sauerstoff enthaltenden Gasen und Cyclohexan wird so aufeinander abgestimmt, daß das aus der Reaktionszone austretende Abgas einen Gehalt an molekularem Sauerstoff von nicht mehr als 2.5. z.B. 0.1 bis 1.5 Vol.%, molekularen Sauerstoff enthält.

Cycloalkanole und Cycloalkanone, die nach dem Verfahren der Erfindung erhalten werden, sind Ausgangsstoffe für die Herstellung von Faserrohstoffen wie Dicarbonsäuren oder Lactamen.

Das Verfahren nach der Erfindung sei an folgenden Beispielen veranschaulicht. Die Beispiele 1,2,3,5,7,8,10,11 und 14 sind Vergleichsbeispiele.

Beispiele 1 bis 7

In einen stehenden zylindrischen Reaktor von 100 l Inhalt und einem Verhältnis von Höhe zu Durchmesser von 2 der keine Einbauten enthält, werden stündlich von unten 155 kg Cyclohexan zugeführt, das 0.3 ppm Kobalt in Form von Kobaltethylhexanoat enthält. Zugleich leitet man stündlich 5 Nm³ Luft zu. Die Luftzuführungsarten werden nachfolgend beschrieben. Die Umsetzung erfolgt bei einer Temperatur von 165°C und unter einem Druck von 15 bar. Am oberen Ende des Reaktors wird Abgas und flüssiges Reaktionsgemisch entnommen. Das flüssige Reaktionsgemisch wird mit Wasser und Natronlauge gewaschen und anschließend das Gemisch aus Cyclohexanol und Cyclohexanon durch Destillation isoliert.

Beispiel 1

Luftzuführung über ein Einsteckrohr von 10 mm Durchmesser ; Austrittsgeschwindigkeit der Luft 1.75 m/sec ; mittlerer Blasendurchmesser 10 bis 40 mm ; Ausbeute 74 Mol.% Cyclohexanol/Cyclohexanon.

Beispiel 2

Luftzuführung über einen Lochboden mit 16 Löchern von 2 mm Durchmesser die gleichmäßig über den Querschnitt verteilt sind ; Austrittsgeschwindigkeit der Luft 2.75 m/sec ; mittlerer Blasendurchmesser 1 bis 10 mm ; Ausbeute 75.5 Mol.% Cyclohexanol/Cyclohexanon.

Beispiel 3

Luftzuführung über einen Lochboden mit 155 Löchern ä 0.5 mm Durchmesser, die über den Querschnitt gleichmäßig verteilt sind ; Austrittsgeschwindigkeit der Luft 4.55 m/sec ; mittlerer Blasendurchmesser 1 bis 6 mm ; Ausbeute 74.5 Mol.% Cyclohexanol/Cyclohexanon.

Beispiel 4

Zuführung der Luft über 16 über den Querschnitt verteilte Düsen nach unten, wobei jede Düse in einer Erweiterung mit einem Durchmesser von 10 mm und einer Höhe von 30 mm endet ; Austrittsgeschwindigkeit der Luft an der Zuführungsstelle in die Flüssigkeit 0.11 m/sec ; mittlerer Blasendurchmesser bei Austritt aus der Düsenöffnung größer 10 mm ; Ausbeute 76 Mol.% Cyclohexanol/Cyclohexanon.

Beispiel 5

Luftzuführung über ein Einsteckrohr von 10 mm Durchmesser. Zusätzlich ist der Reaktor mit einem Scheibenrührer mit einer Umdrehungsgeschwindigkeit von 1 000 Upm und Strombrechern ausgerüstet. Austrittsgeschwindigkeit der Luft 1.75 m/sec : mittlerer Blasendurchmesser 1 bis 2 mm ; Ausbeute 74.5 Mol.% Cyclohexanol/Cyclohexanon.

Beispiel 6

Luftzuführung erfolgt in zwei Ebenen im Abstand von 30 cm über jeweils 16 Bohrungen wie im Beispiel 4 ausgeführt. Austrittsgeschwindigkeit der Luft 0.055 m/sec ; mittlerer Blasendurchmesser bei Austritt aus der Düsenöffnung größer 10 mm ; Ausbeute 77 Mol.% Cyclohexanol/Cyclohexanon.

Beispiel 7

Luftzuführung über eine Zweistoff-Strahldüse mit einem Ringspalt für die Luft von 0.1 mm ; Austrittsgeschwindigkeit der Luft 65.9 m/sec ; mittlerer Blasendurchmesser kleiner 1 mm ; Ausbeute 73 Mol.% Cyclohexanol/Cyclohexanon.

Die Ausbeuten beziehen sich jeweils auf umgesetztes Cyclohexan.

Beispiel 8

Man verfährt wie im Beispiel 1 beschrieben, hält jedoch eine Temperatur von 145°C ein. Der Sauerstoffgehalt im Abgas beträgt 3 bis 4 Vol.%. Die Umsetzung verläuft instabil.

Beispiel 9

Man verfährt wie im Beispiel 4 beschrieben und hält eine Temperatur von 145°C ein. Der Sauerstoffgehalt im Abgas beträgt 0.2 Vol.%. Die Reaktion verläuft stabil.

Beispiele 10 bis 13

In einem zylindrischen Reaktor von 100 l inhalt und einem Verhältnis von Höhe zu Durchmesser von 15, der keine Einbauten enthält, werden von unten stündlich 155 kg Cyclohexan mit einem Gehalt von 0.3 ppm Kobalt in Form von Kobaltethylhexanoat zugeführt. Zugleich leitet man stündlich 5 Nm³ Luft zu. Die Zuführungsarten für die Luft werden nachfolgend ausgeführt. Die Umsetzung wird bei einer Temperatur von 165°C und unter einem Druck von 15 bar durchgeführt.

Die unterschiedlichen Arbeitsweisen gemäß Beispiel 10 bis 13 sowie die erzielten Ergebnisse sind wie folgt :

Beispiel 10

Luftzuführung über ein Einsteckrohr von 10 mit Durchmesser ; Austrittsgeschwindigkeit der Luft 1.75 m/sec : mittlerer Blasendurchmesser bei Austritt aus der Düsenöffnung 10 bis 40 mm : Ausbeute 69 Mol.% Cyclohexanol/Cyclohexanon.

Beispiel 11

Luftzuführung über einen Lochboden mit 16 Löchern ä 2 mm Durchmesser die über den Querschnitt gleichmäßig verteilt sind : Austrittsgeschwindigkeit der Luft 2.75 m/sec : mittlerer Blasendurchmesser 1 bis 10 mm : Ausbeute 71 Mol.% Cyclohexanol/Cyclohexanon.

Beispiel 12

Die Luft über 7 Düsen, die im Abstand von 40 cm im Verlauf des Reaktors nach oben im Querschnitt gesehen, gegeneinander versetzt angeordnet sind. Die Luft wird über Düsen mit Erweiterungen von 10 mm Durchmesser und einer Höhe von 30 mm nach unten zugeführt. Austrittsgeschwindigkeit der Luft 0.22 m/sec ; mittlerer Blasendurchmesser bei Austritt aus der Düsenöffnung größer 10 mm : Ausbeute 73 Mol.% Cyclohexanol/Cyclohexanon.

Beispiel 13

Man verfährt wie in Beispiel 12. Der zylindrische Reaktor ist jeweils zwischen den einzelnen Düsen im Verlauf des Reaktors durch Trennbieche in Kammern abgeteilt. Die Trennbleche enthalten jeweils 16 Löcher von 2 mm Durchmesser. Der freie Querschnitt beträgt, inclusive den Ringspalt am zylindrischen Teil des Reaktors. 2 %. Die Trennbleche sind jeweils unterhalb der Düsen für die Luftzuführung angeordnet. Austritt geschwindigkeit der Luft 0.22 m/sec : mittlerer Blasendurchmesser größer 10 mm : Ausbeute 78 Mol.% Cyclohexanol/Cyclohexanon.

Beispiel 14

In einem Oxidationssystem, bestehend aus drei nacheinander geschalteten Reaktoren (Volumen jeweils 40 m³. Höhe 16 m, Durchmesser 1.8 m). einen Aufarbeitungsteil (Entfernung der Säuren durch Wasserwäsche und Extraktion sowie Neutralisation mit Natronlauge) sowie einen Destillationsteil (Abtrennung des nicht umgesetzten Cyclohexans) wird oxidiert. Die Aufarbeitung und Destillation wird bei den Beispielen 14 und 15 gleichartig betrieben. Die Reaktoren sind jeweils mit einem Umlaufrohr versehen (Höhe 9 m. Durchmesser 1.1 m. 1.0 m oberhalb des Reaktorbodens angeordnet), die Luft und das Cyclohexan werden von unten über zwei konzentrisch angeordnete Rohre eingeleitet. Stündlich werden in das Oxidationssystem 80 t zugepumpt, wobei das Cyclohexan vor jeden Reaktor mit 0.3 ppm Co in Form des Ethylhexansäuresalzes versetzt wird. Die Reaktortemperatur beträgt 150 °C bzw. 145 °C. gemessen am unteren Teil des jeweiligen Reaktors. Der Reaktionsdruck ist 16 bar. In jeden Reaktor werden 1 000 Nm³/h Luft eingeleitet. (Austrittgeschwindigkeit des Gases 0.03 m/sec). Man erhält nach Aufarbeitung eine Ausbeute von 81.2 Mol.% Cyclohexanon und Cyclohexanol, bezogen auf umgesetztes Cyclohexan.

Beispiel 15

In dem gleichen Oxidationssystem wird ein Gasverteiler eingebaut, der die Luft gleichmäßig über den Reaktorquerschnitt und die Reaktorhöhe verteilt. Der Flüssigkeitsinhalt wird in 7 Ebenen in einem Abstand von

5

2 m begast. Zur Verringerung der Ruckvermischung sind im Reaktor jeweils Lochbleche (100 x ∅ 40 mm) mit einer auf den Reaktorquerschnitt bezogenen freien Fläche von 7% 30 cm unterhalb der Gasverteiler eingebaut. Der Gasverteiler jeder Ebene ist derart gestaltet, daß die Luft durch 33 Bohrungen (∅ 3 mm), die gleichmäßig über den Reaktorquerschnitt verteilt sind, an der Unterseite der Begasungsrohre (NW 32) austritt. Jede Bohrung ist mit einem Hüllrohr (L 60 mm. ∅ 25 mm) versehen. Die Gasaustrittgeschwindigkeit unter Betriebsbedingungen ist bei 0.25 m/sec. Das Reaktorsystem wird wie in Beispiel 14 mit 80 t/h Cyclohexan bei 150 °C bzw. 145 °C und 15 bar beaufschlagt. In jeden Reaktor werden 1 000 Nm³/h Luft eingeleitet wobei die Luft gleichmaßig über die Begasungseinrichtung in den Reaktor eingeführt wird. Man erhalt nach Aufarbeitung eine Ausbeute von 86.5 Mol.% Cyclohexanon und Cyclohexanol, bezogen auf umgesetztes Cyclohexan.

## Patentansprüche

1. Verfahren zur kontinuierlichen Herstellung von Sauerstoff enthaltenden Verbindungen durch Oxidation von Kohlenwasserstoffen in flüssiger Phase mit molekularen Sauerstoff enthaltenden Gasen bei erhöhter Temperatur und unter erhöhtem Druck, wobei man die molekularen Sauerstoff enthaltenden Gase an mehreren Stellen im Verlauf der Reaktionszone über Düsenöffnungen nach unten gerichtet einleitet, dadurch gekennzeichnet, daß man die molekularen Sauerstoff enthaltenden Gase mit einer Austrittsgeschwindigkeit an jeder Düsenöffnung von 0,03 bis 0,3 m/sec. in einer Menge von 0,001 bis 10 l/sec. je Düsenöffnung in das flüssige Reaktionsgemisch einleitet und dieses über das Volumen der Reaktionszone im wesentlichen gleichmäßig mit molekularem Sauerstoff enthaltenden Gasen in Berührung bringt.

2. Verfahren nach Anspruch 1, dadurch gekennzeichnet, daß man die flüssigen Kohlenwasserstoffe und die molekularen Sauerstoff enthaltenden Gase im Gleichstrom durch die Reaktionszone leitet.

3. Verfahren nach den Ansprüchen 1 und 2, dadurch gekennzeichnet, daß man die molekularen Sauerstoff enthaltenden Gase im Verlauf der Reaktionszone in Abständen, die dem 0.1 bis 3 fachen des Durchmessers der Reaktionszone entsprechen, jeweils über den Querschnitt der Reaktionszone gleichmäßig verteilte Düsenöffnungen zuführt.

4. Verfahren nach den Ansprüchen 1 bis 3, dadurch gekennzeichnet, daß die Reaktionszone in Kammern unterteiit ist die miteinander in Verbindung stehen.

## Claims

1. A process for the continuous preparation of an oxygen-containing compound by oxidizing a hydrocarbon in the liquid phase with a gas containing molecular oxygen, at elevated temperature and under superatmospheric pressure, the gas containing molecular oxygen being introduced, in a downward direction, at several points along the reaction zone via nozzle orifices, wherein the gas containing molecular oxygen is fed into the liquid reaction mixture, in an amount of from 0.001 to 10 liters per second per nozzle orifice, the velocity at which said gas emerges from each nozzle orifice being from 0.03 to 0.3 m/sec, and said reaction mixture is brought into substantially uniform contact with the gas containing molecular oxygen, over the volume of the reaction zone.

2. A process as claimed in claim 1, wherein the liquid hydrocarbon and the gas containing molecular oxygen are passed cocurrently through the reaction zone.

3. A process as claimed in claim 1 or 2, wherein the gas containing molecular oxygen is fed to nozzle orifices which are uniformly distributed along the reaction zone at intervals of from 0.1 to 3 times the diameter of this zone, and over the cross-section of the particular reaction zone.

4. A process as claimed in any of claims 1 to 3, wherein the reaction zone is divided into chambers which communicate with one another.

## Revendications

1. Procédé de préparation continue de composés contenant de l'oxygène, par oxydation d'hydrocarbures, en phase liquide,avec des gaz contenant de l'oxygène moléculaire, à température élevée et sous pression élevée, en introduisant, dirigés vers le bas, par des orifices de tuyères,les gaz contenant l'oxygène moléculaire en plusieurs endroits, dans le cours de la zone de réaction, caractérisé par le fait qu'on introduit les gaz contenant de l'oxygène moléculaire, à une vitesse de sortie à chaque orifice de tuyère de 0,03 à 0,3 m/s, en une quantité de 0,001 à 10 l/s, dans le mélange de réaction liquide et on met celui-ci en contact, sur le volume de la zone de réaction, en principe régulièrement, avec des gaz contenant de l'oxygène moléculaire.

2. Procédé selon la revendication 1, caractérisé par le fait que l'on fait passer les hydrocarbures liquides et les gaz contenant de l'oxygène moléculaire à travers la zone de réaction en courants de même sens.

3. Procédé selon les revendications 1 et 2, caractérisé par le fait que l'on envoie les gaz contenant de l'oxygène moléculaire, à intervalles correspondant à 0,1 à 3 fois le diamètre de la zone de réaction, à des orifices de tuyères répartis régulièrement sur la section de la zone de réaction.

4. Procédé selon les revendications 1 à 3, caractérisé par le fait que la zone de réaction est subdivisée en chambres qui sont reliées les unes aux autres.